(19) Europäisches Patentamt / European Patent Office / Office européen des brevets

(11) **EP 3 028 626 A1**

(12) **EUROPEAN PATENT APPLICATION**

(43) Date of publication:
**08.06.2016 Bulletin 2016/23**

(51) Int Cl.:
**A61B 3/10** *(2006.01)*  **G02B 7/182** *(2006.01)*
**G02B 26/08** *(2006.01)*

(21) Application number: **15198061.2**

(22) Date of filing: **04.12.2015**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **04.12.2014 JP 2014246345**
**06.11.2015 JP 2015218782**

(71) Applicant: **Canon Kabushiki Kaisha**
**Tokyo 146-8501 (JP)**

(72) Inventors:
• **TAKAHAMA, Shinichiro**
**Ohta-ku, Tokyo (JP)**
• **TAMAMORI, Kenji**
**Ohta-ku, Tokyo (JP)**

(74) Representative: **Houle, Timothy James**
**Canon Europe Ltd**
**European Patent Department**
**3 The Square**
**Stockley Park**
**Uxbridge, Middlesex UB11 1ET (GB)**

(54) **DEFORMABLE MIRROR, OPTICAL SYSTEM INCLUDING THE DEFORMABLE MIRROR, AND OPHTHALMOLOGIC APPARATUS**

(57) A mount substrate (third substrate) (300) is disposed so as to face a mirror substrate (second substrate) (200) having a larger projection area, and the mirror substrate (200) is bonded to the mount substrate (300) in a region in which an actuator substrate (100) and the mirror substrate (200) do not overlap each other in an in-plane direction (XY direction) parallel to an in-plane direction of a reflective member (202).

# FIG. 3A

EP 3 028 626 A1

**Description**

BACKGROUND OF THE INVENTION

Field of the Invention

The present invention relates to deformable mirrors, optical systems including the deformable mirrors, and ophthalmologic apparatuses.

Description of the Related Art

Deformable mirrors that are deformed by an electrostatic attraction or an electromagnetic force are expected to find applications in a variety of fields in which light is used. For example, such a deformable mirror can be used as a wavefront correction device in an adaptive optical system for a funduscopy apparatus, an astronomical telescope, or the like.

U.S. Patent No. 6,384,952 discloses a deformable mirror in which actuators constituted by comb electrodes are bonded to a membrane mirror. Each actuator includes a plurality of movable comb electrodes and a plurality of fixed comb electrodes, and the plurality of movable comb electrodes and the plurality of fixed comb electrodes are disposed in an alternating manner in an in-plane direction with a gap provided therebetween. The movable comb electrodes in the respective actuators are moved separately in a direction perpendicular to the in-plane direction, and thus the shape of the membrane mirror can be controlled.

Meanwhile, an actuator substrate provided with the actuators is mounted on a mount substrate that is provided with a drive circuit for driving the actuators, either directly or indirectly with another substrate or the like affixed therebetween.

Bonding portions that bond the actuators to the membrane mirror as in those disclosed in U.S. Patent No. 6,384,952 are very small in size as compared to the actuator substrate and the membrane mirror. Thus, if the bonding portions are pressurized when the actuator substrate is affixed to the mount substrate, some of the bonding portions bear a load and may deform, which may cause breakage to occur.

SUMMARY OF THE INVENTION

The present invention is directed to suppressing deformation of such bonding portions in a deformable mirror provided with a mount substrate.

The present invention in its first aspect provides a method for fabricating a deformable mirror as specified in claims 1 to 4.

The present invention in its second aspect provides a deformable mirror as specified in claims 5 to 14.

Further features of the present invention will become apparent from the following description of exemplary embodiments with reference to the attached drawings.

BRIEF DESCRIPTION OF THE DRAWINGS

Figs. 1A, 1B, 1C schematically illustrate an example of a deformable mirror according to a first exemplary embodiment.

Figs. 2A and 2B are illustrations for describing an issue.

Figs. 3A and 3B schematically illustrate an example of a process of bonding a mirror substrate to a mount substrate according to the first exemplary embodiment.

Figs. 4A, 4B, and 4C schematically illustrate an example of a configuration of an actuator substrate and actuators in a deformable mirror according to the first exemplary embodiment.

Figs. 5A, 5B, 5C, and 5D are illustrations for describing the driving of the actuators.

Figs. 6A, 6B, 6C, 6D, 6E, 6F, 6G, and 6H schematically illustrate an example of a method for fabricating an actuator and a process of bonding the actuator to a mirror substrate according to the first exemplary embodiment.

Figs. 7A and 7B schematically illustrate an example of a process of exposing a reflective member according to the first exemplary embodiment.

Figs. 8A, 8B, and 8C schematically illustrate an example of a deformable mirror according to second and third exemplary embodiments.

Figs. 9A and 9B schematically illustrate an example of a process of bonding a mirror substrate to a mount substrate according to the second exemplary embodiment.

Figs. 10A and 10B schematically illustrate examples of an actuator substrate and a mirror substrate, respectively, according to the second and third exemplary embodiments.

Figs. 11A and 11B schematically illustrate an example of a deformable mirror according to a fourth exemplary embodiment.

Fig. 12 schematically illustrates an example of an ophthalmologic apparatus according to a fifth exemplary embodiment.

DESCRIPTION OF THE EMBODIMENTS

A deformable mirror according to some exemplary embodiments of the present invention will be described in detail with reference to the drawings. It is to be noted that the present invention is not limited to the configurations of these exemplary embodiments.

First Exemplary Embodiment

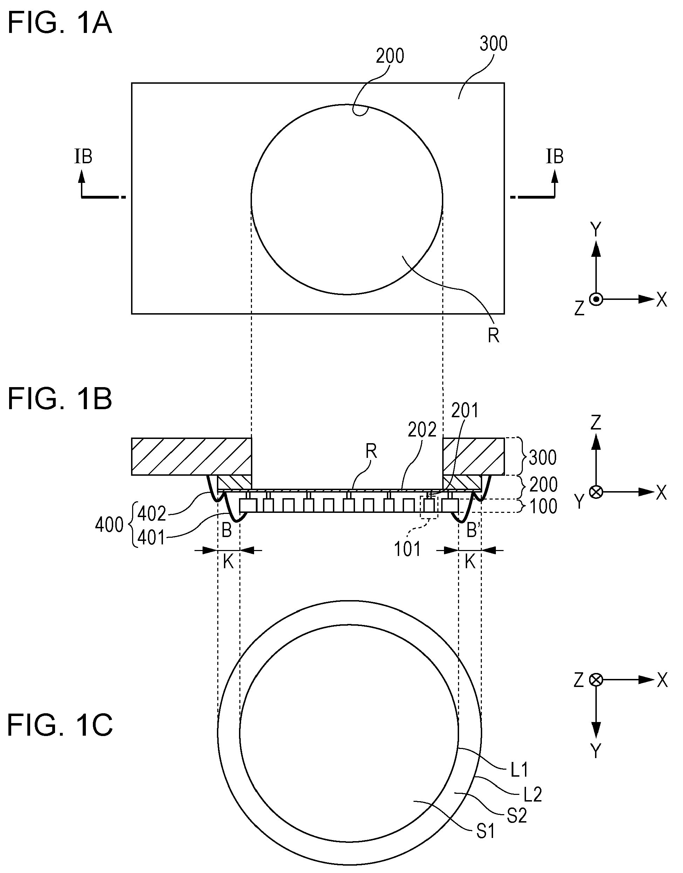

Figs. 1A to 1C schematically illustrate a deformable mirror according to the present exemplary embodiment. Fig. 1A schematically illustrates the deformable mirror as viewed from a side on which a reflective surface R of a reflective member is exposed (+Z direction). Fig. 1B is a schematic sectional view of the deformable mirror taken along the line IB-IB indicated in Fig. 1A. The deformable mirror includes an actuator substrate (first substrate) 100 provided with a plurality of actuators 101 and a mirror substrate (second substrate) 200 provided with a reflective member 202, which is bonded to the plurality of actuators 101 with bonding portions 201 interposed therebetween. The deformable mirror further includes a mount substrate (third substrate) 300 provided with a drive circuit for driving the plurality of actuators 101.

Fig. 1C illustrates a relationship between the areas defined by the respective outer peripheries of the shadows of the actuator substrate 100 and the mirror substrate 200 obtained when the shadows are projected on a plane (XY plane) that is parallel to the in-plane direction of the reflective member 202 (hereinafter, referred to as the projection areas). In the present exemplary embodiment, the projection area S1 of the actuator substrate 100 is smaller than the projection area S2 of the mirror substrate 200. The shadow of the actuator substrate 100 projected on the plane (XY plane) parallel to the in-plane direction of the reflective member 202 has an outer periphery L1, and the shadow of the mirror substrate 200 projected on the plane (XY plane) parallel to the in-plane direction of the reflective member 202 has an outer periphery L2.

As illustrated in Fig. 1B, the mount substrate 300 is disposed so as to face the mirror substrate 200 that has a larger projection area than the actuator substrate 100. The mount substrate 300 is bonded to the mirror substrate 200 in a region K in which the mirror substrate 200 and the actuator substrate 100 do not overlap each other in an in-plane direction parallel to the in-plane direction of the reflective member 202. The effect of this configuration will be described hereinafter.

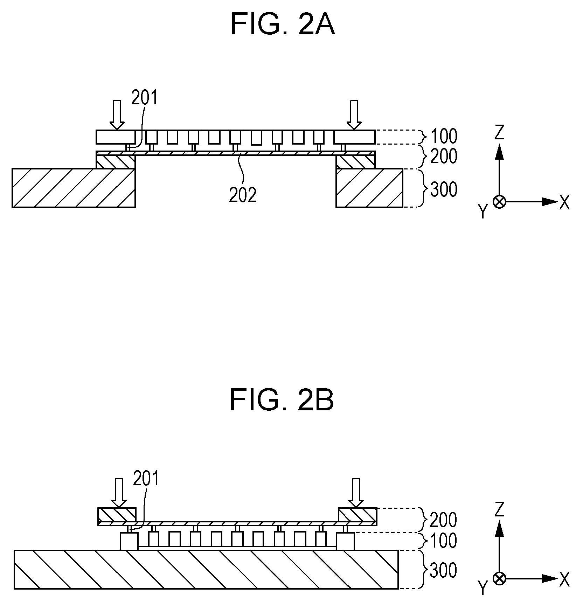

Fig. 2A illustrates a process of bonding the mirror substrate 200 to the mount substrate 300 in a case in which the projection area of the actuator substrate 100 is equal to the projection area of the mirror substrate 200. In this process, due to the constraints of a fabrication device, an external force is exerted on the actuator substrate 100 in order to bond the mirror substrate 200 to the actuator substrate 100 and to the mount substrate 300. The direction of the external force is indicated by the arrows in Fig. 2A. In the stated case, some of the external force is also transmitted to the bonding portions 201 disposed between the actuator substrate 100 and the reflective member 202. Typically, the bonding area on each bonding portion 201 (the width of each bonding portion 201) bonded to an actuator 101 or the reflective member 202 is no less than one-ten-thousandth and no greater than one-hundredth of the projection area of the mirror substrate 200, and the bonding portions 201 are very small. In other words, the bonding portions 201 have low mechanical strength. Therefore, the bonding portions 201 deform due to the external force. This deformation may prevent the driving of the actuators 101 from being transmitted to the reflective member 202 at a sufficient level or may cause breakage during the driving. In a similar manner, an external force is exerted on the bonding portions 201 when the actuator substrate 100 is to be bonded to the mount substrate 300, and a similar problem may arise.

A case in which the projection area of the mirror substrate 200 is greater than the projection area of the actuator substrate 100 will now be considered. Fig. 2A illustrates a process of bonding the mount substrate 300 to the actuator substrate 100 that has a smaller projection area than the mirror substrate 200. In this configuration, the mirror substrate 200, which has a greater projection area, is pressurized in order to bond the mirror substrate 200 to the actuator substrate 100 and to the mount substrate 300. Thus, an external force is exerted on the bonding portions 201, and a problem similar to the one described with reference to Fig. 2A arises. A similar problem also arises in a case in which the projection area of the actuator substrate 100 is greater than the projection area of the mirror substrate 200 and the mount substrate 300 is to be bonded to the mirror substrate 200 that has a smaller projection area than the actuator substrate 100.

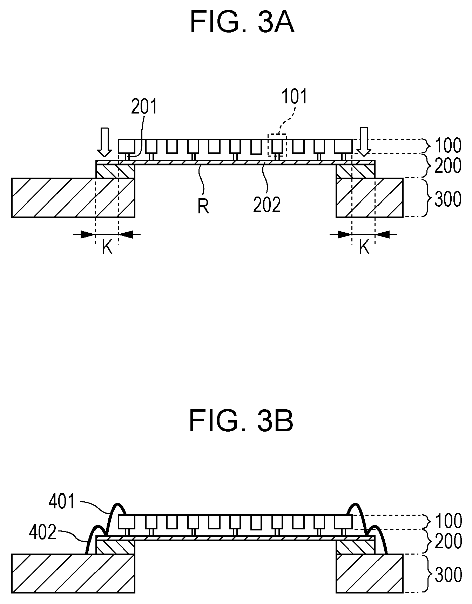

In the meantime, as illustrated in Fig. 3A, in the present exemplary embodiment, the mount substrate 300 is bonded to the mirror substrate 200 that has a greater projection area than the actuator substrate 100. In this configuration, in order to bond the mirror substrate 200 to the actuator substrate 100 and to the mount substrate 300, an external force can be exerted on the mirror substrate 200 in a region in which the mirror substrate 200 is directly bonded to the mount substrate 300. Specifically, the external force can be exerted (as indicated by the arrows) in the region K in which the mirror substrate 200 and the actuator substrate 100 do not overlap each other in an in-plane direction parallel to the in-plane direction of the reflective member 202. Thus, the mirror substrate 200 can be bonded to the mount substrate 300 without the external force being exerted on the bonding portions 201. Accordingly, deformation of the bonding portions 201 can be suppressed.

As illustrated in Fig. 1B, the mirror substrate 200 includes the reflective member 202 having an optical reflection function of reflecting light to be corrected. Of the two surfaces of the reflective member 202, the one on the upper side in the paper plane serves as the reflective surface R. The reflective member 202 is configured to cover the actuator substrate 100. The mirror substrate 200 is constituted by a silicon-on-insulator (SOI) substrate, and with a handle layer (Si) and a box layer (silicon oxide) having been removed, the reflective member 202 formed by an active layer (Si) is exposed. The reflective member 202 is provided with the bonding portions 201 to be bonded to the actuators 101. In the reflective member 202, the reflective surface may be formed as a metal film of gold or the like is deposited in the region in which the handle layer and the box layer have been removed. The reflective member 202 is constituted by a thin film so that its shape can be changed by the actuators 101. Specifically, the reflective member 202 has a thickness that falls within a range from no less than 500 nm to no greater than 3 $\mu$m.

As illustrated in Fig. 1B, the mount substrate 300 is disposed on the side of the mirror substrate 200 where light is incident on the reflective member 202 and reflected thereby. Thus, the mount substrate 300 has an opening through which the reflective member 202 is exposed. In addition, the mount substrate 300 includes a drive circuit (not illustrated) that is electrically connected to the actuators 101 with a conductive member (not illustrated) formed in the mirror substrate 200 interposed therebetween. Specifically, the drive circuit and the actuators 101 are electrically connected to each other by bonding wires 400. To be more specific, the actuators 101 and the conductive member in the mirror substrate 200 are electrically connected to each other by first bonding wires 401. The conductive member, to which the first bonding wires 401 are electrically connected, in the mirror substrate 200 and the drive circuit of the mount substrate 300 are electrically connected to each other by second bonding wires 402.

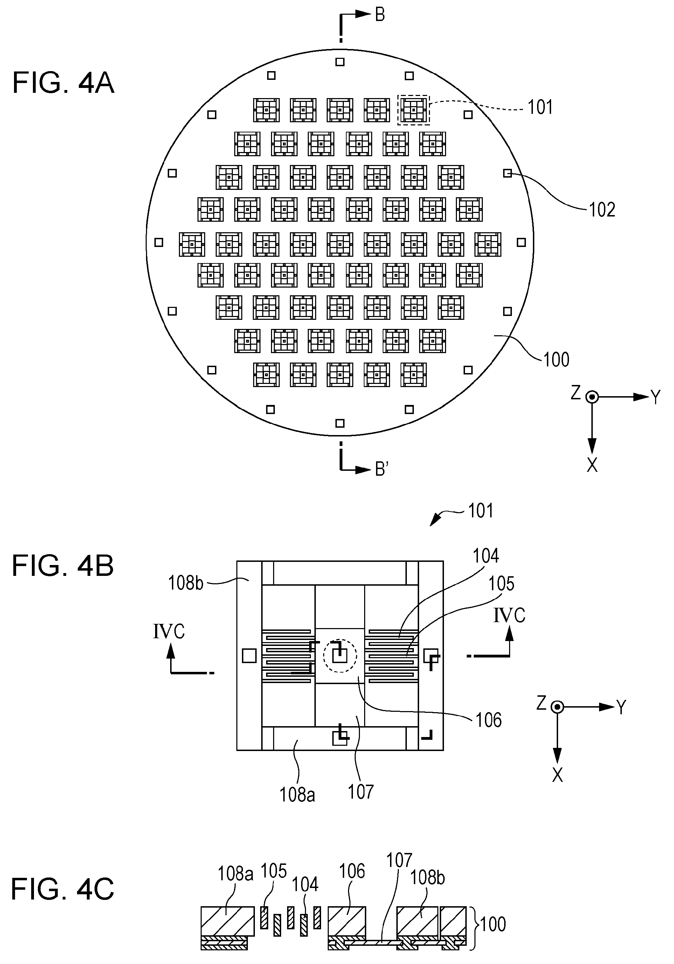

The actuator substrate 100 will be described with reference to Figs. 4A to 4C. Fig. 4A schematically illustrates the actuator substrate 100 as viewed in the +Z direction, with the mirror substrate 200 and the mount substrate 300 omitted. A sectional view taken along the line B-B' in Fig. 4A corresponds to the sectional view illustrated in Fig. 1B. The actuator substrate 100 includes the plurality of actuators 101. The actuators 101 are bonded to the reflective member 202 of the mirror substrate 200 with the bonding portions 201 interposed therebetween. In addition, the actuator substrate 100 is bonded to the reflective member 202 in bonding regions 102 with the bonding portions 201 interposed therebetween. The bonding regions 102 may be located so as to correspond to regions other than the region where the reflective member 202 is exposed.

Fig. 4B illustrates one of the actuators 101 as viewed in the +Z direction, and Fig. 4C is a schematic sectional view of the actuator 101 taken along the line IVC-IVC indicated in Fig. 4B.

Each actuator 101 includes movable comb electrodes 104, fixed comb electrodes 105, a movable portion 106, spring portions 107, and support portions 108a and 108b. The movable portion 106 is linked to one end of each spring portion 107 and connected to the movable comb electrodes 104 and the reflective member 202. The other end of each spring portion 107 is fixed to a corresponding support portion 108a. The movable comb electrodes 104 and the spring portions 107 are connected to the side walls of the movable portion 106, and the reflective member 202 (see Fig. 1B) is bonded to the upper surface, which has a relatively large area, of the movable portion 106. Specifically, the upper surface of the movable portion 106 is bonded to the back surface of the reflective member 202 that is on the opposite side of the reflective surface R (see Fig. 1B). The spring portions 107 function as a restraining unit that allows the movable comb electrodes 104 and the movable portion 106 to be displaced in a direction normal to the reflective surface R (+Z direction) but restrains the movable comb electrodes 104 and the movable portion 106 from being displaced in directions other than the normal direction. Although the restraining unit is constituted by the elastic spring portion 107 herein, the restraining unit can also be constituted by a guide unit that guides the movable portion 106 by allowing the movable portion 106 to be displaced in the normal direction but restraining the movable portion 106 from being displaced in directions other than the normal direction. The support portions 108a, which are electrically connected to the movable comb electrodes 104, are insulated from the support portions 108b, which are electrically connected to the fixed comb electrodes 105, by insulating portions formed at boundaries between the support portions 108a and 108b.

The movable comb electrodes 104 extend in the Y direction from the side walls of the movable portion 106 that are parallel to the XZ plane, and the fixed comb electrodes 105 extend in the Y direction from the side walls of the support portions 108b that are parallel to the XZ plane. Specifically, the movable comb electrodes 104 are spaced apart from the reflective member 202 (see Fig. 1B) and supported by the movable portion 106 so as to extend in the direction parallel to the reflective surface. Meanwhile, the fixed comb electrodes 105 are supported by the support portions 108b so as to extend in the direction parallel to the reflective surface and are disposed so as to alternate with the movable comb electrodes 104 with a gap provided therebetween. Since the side walls of the movable portion 106 face the side walls of the support portions 108b, the movable comb electrodes 104 and the fixed comb electrodes 105 are disposed so as to face each other and are disposed such that their comb elements alternate with each other. In other words, the sections of the movable portion 106 that support the movable comb electrodes 104 and the sections of the support portions 108b that support the fixed comb electrodes 105 are disposed so as to allow the movable comb electrodes 104 to be displaced with the gap between the movable comb electrodes 104 and the fixed comb electrodes 105 retained. The movable comb electrodes 104 and the fixed comb electrodes 105 are disposed such that a difference in their levels is produced in the Z direction.

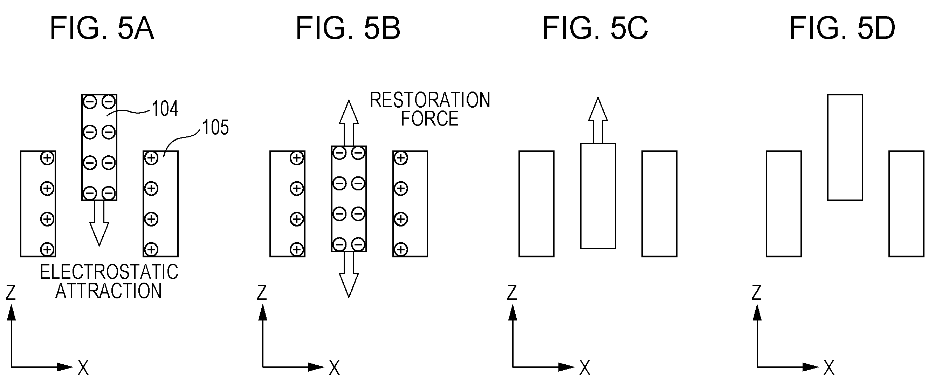

A method for driving the movable portion 106 of the actuator 101 will now be described with reference to Figs. 5A to 5D. Figs. 5A to 5D are sectional views of a portion where the movable comb electrodes 104 and the fixed comb electrodes 105 are arranged in an alternating manner. Electric charges with opposite signs are given to the movable comb electrodes 104 and the fixed comb electrodes 105, and thus the movable comb electrodes 104 can be moved in the Z direction. An electrostatic attraction Fz that acts in the Z direction when a potential difference is given between the movable comb electrodes 104 and the fixed comb electrodes 105 is expressed through the following expression (1).

$$F_z = \frac{\varepsilon_0 Nh}{2g}(V_m - V_f)^2 \qquad \cdots (1)$$

Here, $\varepsilon_0$ represents the dielectric constant of vacuum; N represents the number of gaps between the comb electrodes; h represents an overlap length of the movable comb electrodes 104 and the fixed comb electrodes 105; $V_m$ represents the potential of the movable comb electrodes 104; $V_f$ represents the potential of the fixed comb electrodes; and g represents the width of the gap between the comb electrodes.

For example, a possible method for moving the movable comb electrodes 104 in the -Z direction when the movable comb electrodes 104 and the fixed comb electrodes 105 are disposed as illustrated in Figs. 5A to 5D is as follows. As seen in Fig. 5A depicting a state immediately after a voltage has been applied, electric charges with opposite signs are first given to the movable comb electrodes 104 and the fixed comb electrodes 105, which causes an electrostatic attraction to be generated, and the electrodes attract each other. Thus, the movable comb electrodes 104 are pulled toward the fixed comb electrodes 105, but the electrostatic attraction acts substantially equally toward the right and left along the X-direction, and the movable comb electrodes 104 are thus displaced in the -Z direction. Along with this displacement, the movable portion 106 connected to the movable comb electrodes 104 is displaced, and the region of the reflective member 202 that is connected to the movable portion 106 with the bonding portion 201 interposed therebetween is displaced.

Subsequently, a balanced state as illustrated in Fig. 5B is obtained. Specifically, the movable comb electrodes 104 stop at a position where the restoration force of the spring portions 107 (see Figs. 4B and 4C) and the electrostatic attraction that has moved the movable portion 106 are in balance.

When the potential difference between the movable comb electrodes 104 and the fixed comb electrodes 105 is brought to 0, a state in which electric charges are not given as illustrated in Fig. 5C is obtained. Upon the voltage being released, the movable comb electrodes 104 return to the initial position due to the restoration force of the spring portions 107 (see Figs. 4B and 4C). The state obtained after this displacement is illustrated in Fig. 5D. Although the displacement caused by the electrostatic attraction is described in the present exemplary embodiment, the displacement can also be achieved by an electrostatic repulsion.

In this manner, the shape of the reflective member 202 can be changed by displacing the regions of the reflective member 202 that are bonded to the respective actuators 101 while adjusting the amount of movement of the movable portions 106 of the respective actuators 101.

The amount of movement can be estimated by measuring the capacitance and can thus be controlled by feedback control. The actuators 101 may be driven in a vacuum or in the air.

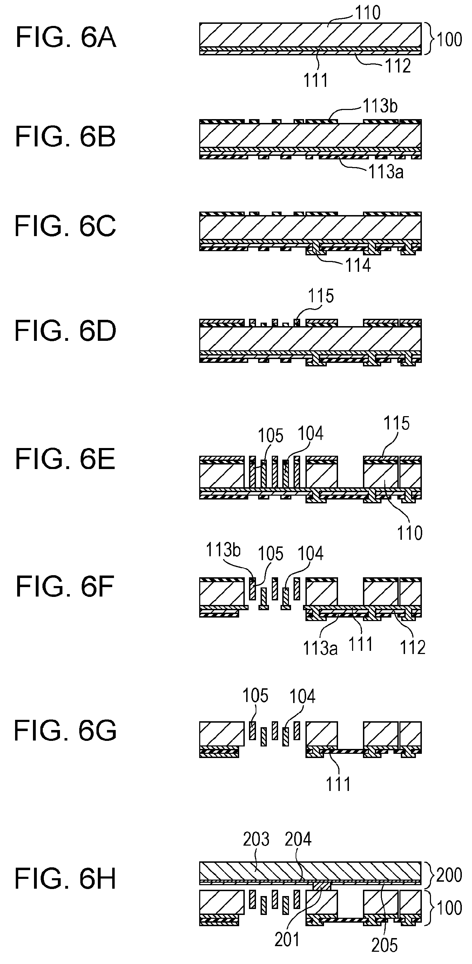

A method for fabricating the actuators 101 according to the present exemplary embodiment will be described with reference to Figs. 6A to 6H, with the examples of specific materials and numerical values provided. Figs. 6A to 6H are sectional views taken along the IVC-IVC line indicated in Fig. 4B.

As illustrated in Fig. 6A, the actuator substrate 100 is prepared first (S101). The actuator substrate 100 is constituted by an SOI substrate. The SOI substrate includes a handle layer (Si) 110 having a thickness of 525 $\mu$m, a box layer (silicon oxide) 111 having a thickness of 1 $\mu$m, and an active layer (Si) 112 having a thickness of 1 $\mu$m. The actuator substrate 100 has dimensions of 10 mm (longitudinal) by 20 mm (lateral).

Subsequently, as illustrated in Fig. 6B, patterns of insulating layers 113a and 113b are formed on respective surfaces of the actuator substrate 100 (S102). Thermally oxidized silicon oxide (silicon oxide) serving as the insulating layers 113a and 113b is formed, and then resist patterns (not illustrated) are formed. A process of etching the insulating layers 113a and 113b with the resist patterns (not illustrated) serving as masks is carried out. For example, in the etching, plasma etching by a chlorofluorocarbon-based gas, such as tetrafluoromethane ($CF_4$), difluoromethane ($CH_2F_2$), or trifluoromethane ($CHF_3$), is employed. These chlorofluorocarbon-based gases can be used individually or can be mixed with another chlorofluorocarbon gas or an inert gas, such as argon (Ar) or helium (He), and the mixed gas can be used.

Next, as illustrated in Fig. 6C, through-electrodes 114 to be electrically connected to the respective support portions 108b (see Fig. 4B) are formed (S103). A resist pattern (not illustrated) is formed on the back surface of the actuator substrate 100. With the resist pattern (not illustrated) serving as a mask, the active layer (Si) 112 and the box layer (silicon oxide) 111 are etched so as to form through-holes. Titanium (Ti) and gold (Au) to serve as electrode materials are deposited to form the layers, and a resist pattern (not illustrated) is then formed. With the resist pattern (not illustrated) serving as a mask, gold (Au) and titanium (Ti) are etched.

Subsequently, as illustrated in Fig. 6D, a mask for forming the comb elements is formed (S104). A resist pattern 115 is formed on the surface of the actuator substrate 100, and the insulating layer 113b on the surface of the actuator substrate 100 is etched. The insulating layer 113b is etched through plasma etching with the use of the chlorofluorocarbon-based gas illustrated in S102.

Subsequently, as illustrated in Fig. 6E, the movable comb electrodes 104 and the fixed comb electrodes 105 are formed from the surface of the actuator substrate 100 (S105). A process of etching the handle layer (Si) 110 with the resist pattern 115 formed in S104 and the insulating layer 113b serving as masks is carried out. In order to form the comb elements with a desired shape by etching the handle layer (Si) 110, inductively coupled plasma-reactive ion etching (ICP-RIE) or the like that enables etching with high profile verticality is used. With the use of ICP-RIE, a fine comb element structure with a high aspect ratio can be formed. Here, a groove that is to serve as an insulating portion is also formed in the handle layer 110.

Subsequently, as illustrated in Fig. 6F, a difference between the levels of the comb elements is produced (S106). In order to produce the difference of the level of the fixed comb electrodes 105 from that of the movable electrodes 104, the active layer (Si) 112 and the box layer (silicon oxide) 111 are etched with the insulating layer (silicon oxide) 113a on the back surface serving as a mask. Furthermore, silicon (Si) of the fixed comb electrodes 105 is etched. Meanwhile, in order to produce the difference of the level of the movable comb electrodes 104 from that of the fixed comb electrodes 105, the resist pattern 115 on the front surface and the resist pattern (not illustrated) on the back surface are peeled, and silicon (Si) of the movable comb electrodes 104 is then etched with the insulating layer (silicon oxide) 113b on the front surface serving as a mask. The silicon (Si) layer and the insulating layer are etched through plasma etching with the use of the chlorofluorocarbon-based gas illustrated in S102 or through ICP-RIE illustrated in S104. Here, the fixed comb electrodes 105 and the movable comb electrodes 104 are formed at the same time by etching the actuator substrate 100. Then, the difference between the levels of the fixed comb electrodes 105 and the movable comb electrodes 104 in the vertical direction of the paper plane is produced.

Subsequently, as illustrated in Fig. 6G, the box layer (silicon oxide) 111 is etched (S107). In etching the box layer (silicon oxide) 111, the box layer (silicon oxide) 111 is selectively wet-etched with 0.5% hydrofluoric acid (HF). In order to selectively etch the box layer (silicon oxide) 111, aside from the hydrofluoric acid, any aqueous solution that contains fluorine ions, such as an aqueous solution of ammonium fluoride ($NH_4F$) or a mixed solution of hydrogen fluoride and hydrogen peroxide, can be used.

A process of bonding the actuator substrate 100 to the mirror substrate 200 will now be described with reference to Fig. 6H. As illustrated in Fig. 6H, the actuator substrate 100 formed through the processes up to S107 is bonded to the mirror substrate 200 (S108). The mirror substrate 200 is constituted by an SOI substrate. The SOI substrate includes a handle layer (Si) 203 having a thickness of 525 $\mu$m, a box layer (silicon oxide) 204 having a thickness of 1 $\mu$m, and an active layer (Si) 205 having a thickness of 1 $\mu$m. The mirror substrate 200 has dimensions of 32 mm (longitudinal) by 32 mm (lateral).

Prior to the bonding process, the mirror substrate 200 is subjected to the following treatment. First, an insulating layer (not illustrated) of thermally oxidized silicon oxide is formed on the surface of the mirror substrate 200. Then, a resist pattern (not illustrated) is formed, and the insulating layer is patterned through wet-etching as illustrated in S107. Subsequently, a resist pattern (not illustrated) is formed on the active layer 205 of the mirror substrate 200, and a post that is to serve as the bonding portion 201 is formed through plasma etching with the use of the chlorofluorocarbon-based gas illustrated in S102.

The actuator substrate 100 can be bonded to the mirror substrate 200 through silicon-silicon (Si-Si) fusion bonding or the like. The advantages of the fusion bonding include high positional precision of the bonding with respect to the vertical direction of the paper plane, which is the direction in which the movable portion 106 can be moved, and that a separate member is not necessary. Meanwhile, bump bonding, which allows the bonding to be carried in a low-temperature process, or bonding with an adhesive can also be employed.

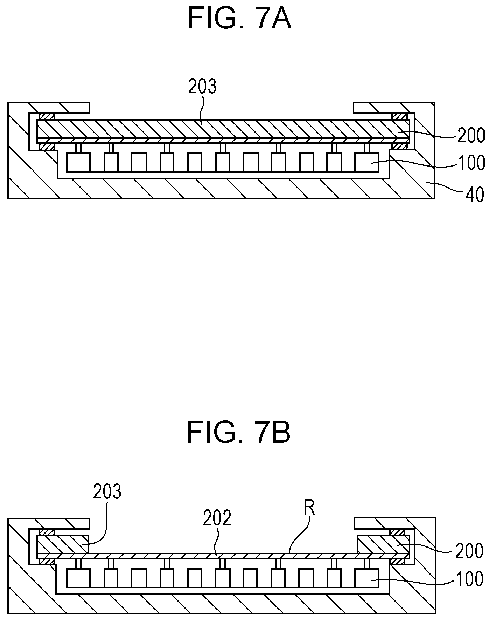

A process of exposing the reflective surface R of the reflective member 202 will now be described with reference to Figs. 7A and 7B. First, as illustrated in Fig. 7A, in order to protect the actuator substrate 100, a jig 40 is used to provide a seal so that an etching liquid does not enter through the edge portion of the mirror substrate 200 that has a larger projection area than the actuator substrate 100.

Subsequently, as illustrated in Fig. 7B, the handle layer (Si layer) 203 and the box layer (silicon oxide) (not illustrated) of the mirror substrate 200 are selectively etched (S109). In order to selectively etch the handle layer (Si) 203, a medical fluid, such as a tetramethylammonium hydroxide aqueous solution (TMAH) or potassium hydroxide (KOH), can be used. The exposed box layer (silicon oxide) can be selectively etched through wet-etching as illustrated in S107. Through this process, the active layer (Si) that is to serve as the reflective member 202 is exposed, and the reflective surface R is exposed.

Subsequently, a method for mounting the mount substrate 300 will be described with reference to Figs. 3A and 3B. The mount substrate 300 includes the drive circuit for supplying electricity to the electrodes in each of the actuators 101 on the actuator substrate 100. The mount substrate 300 applies a specified voltage-100 V in this example-independently to the electrodes in each of the sixty-one actuators 101. The mount substrate 300 is electrically connected to the actuator substrate 100 with the mirror substrate 200 interposed therebetween, and thus wires need to be provided between the actuator substrate 100 and the mount substrate 300 in a number equal to the number of the drive electrodes in the sixty-one actuators 101. Here, wiring is provided by Au wires in two steps between an electrode pad in the actuator substrate 100 and an electrode pad in the mirror substrate 200 and between the electrode pad in the mirror substrate 200 and an electrode pad in the mount substrate 300, and thus electricity is supplied from the mount substrate 300 to the actuator substrate 100. However, the method for wiring is not limited to the above-described method, and the electrical connection may be achieved with a reduced number of Au wires by wiring through the mirror substrate 200.

First, the actuator substrate 100 provided with the plurality of actuators 101 and the mirror substrate 200 provided with the reflective member 202 are prepared, and the actuator substrate 100 and the mirror substrate 200 are bonded to each other with the bonding portions 201 interposed therebetween. As described above, this preparation process may include a process of bonding the actuator substrate 100 provided with the plurality of actuators 101 to the mirror substrate 200 provided with the reflective member 202 with the bonding portions 201 interposed therebetween. In addition, this preparation process may include a process of obtaining, through purchase or the like, the actuator substrate 100 provided with the plurality of actuators 101 and the mirror substrate 200 provided with the reflective member 202 and bonded to the actuator substrate 100 with the bonding portions 201 interposed therebetween.

The mount substrate 300 has an opening having a diameter of 20 mm to allow the reflective surface R of the reflective member 202 to be exposed therethrough. Then, as illustrated in Fig. 3A, the mirror substrate 200 and the mount substrate 300 are positioned and bonded to each other such that the reflective surface R of the reflective member 202 is exposed through the aforementioned opening. As described above, the mirror substrate 200 is bonded to the mount substrate 300 by having an external force exerted on the mirror substrate 200 in the region K in which the mirror substrate 200 and the actuator substrate 100 do not overlap each other in an in-plane direction parallel to the in-plane direction of the reflective member 202. Any desired adhesive can be used to achieve this bonding. The mount substrate 300 is constituted by a glass epoxy substrate having dimensions of 130 mm (longitudinal) by 70 mm (lateral) by 1.6 mm (thickness).

Then, as illustrated in Fig. 3B, wire bonding is provided by the bonding wires 401 between the Au pad (not illustrated) electrically connected to the actuators 101 on the actuator substrate 100 and the Au pad (not illustrated) in the mirror substrate 200. Furthermore, wire bonding is provided by the bonding wires 402 between the Au pad in the mirror substrate 200 and the Au pad electrically connected to the drive circuit of the mount substrate 300. In this manner, the mount substrate 300 is mounted, and the deformable mirror is fabricated.

Second Exemplary Embodiment

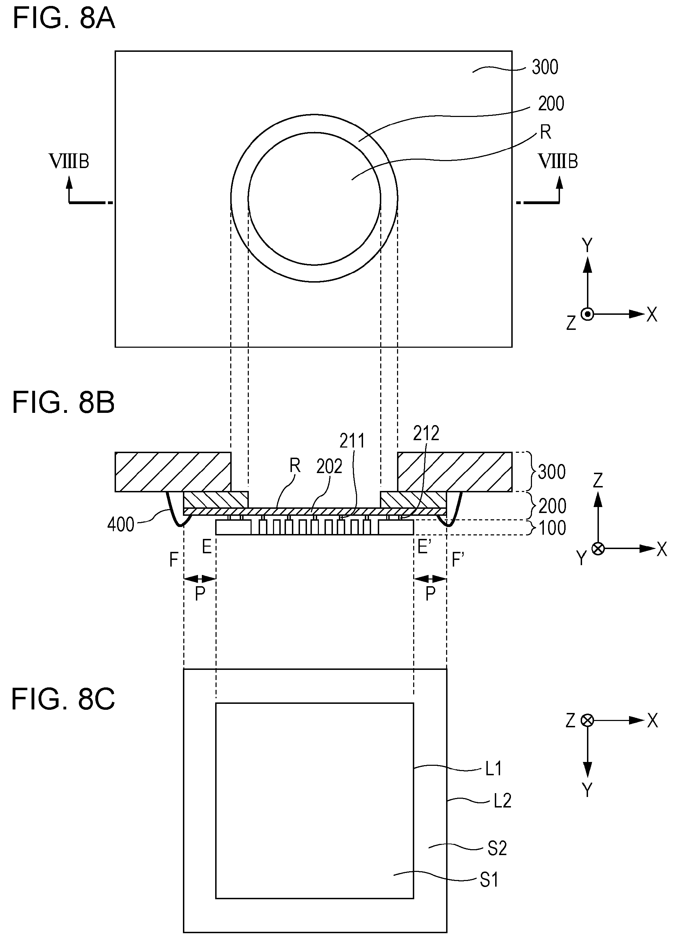

Figs. 8A to 8C schematically illustrate a deformable mirror according to the present exemplary embodiment. Fig. 8A schematically illustrates the deformable mirror as viewed from a side on which the reflective surface R of the reflective member is exposed (+Z direction). Fig. 8B is a schematic sectional view of the deformable mirror taken along the VIIIB-VIIIB line indicated in Fig. 8A. Fig. 8C illustrates a relationship between the areas defined by the respective outer peripheries of the shadows of the actuator substrate 100 and the mirror substrate 200 obtained when the shadows are projected on a plane (XY plane) parallel to the in-plane direction of the reflective member 202 (hereinafter, referred to as the projection areas). In the present exemplary embodiment, as in the first exemplary embodiment, the projection area S1 of the actuator substrate 100 is smaller than the projection area S2 of the mirror substrate 200. The shadow of the actuator substrate 100 projected on the plane (XY plane) parallel to the in-plane direction of the reflective member 202 has an outer periphery L1, and the shadow of the mirror substrate 200 projected on the plane (XY plane) parallel to the in-plane direction of the reflective member 202 has an outer periphery L2.

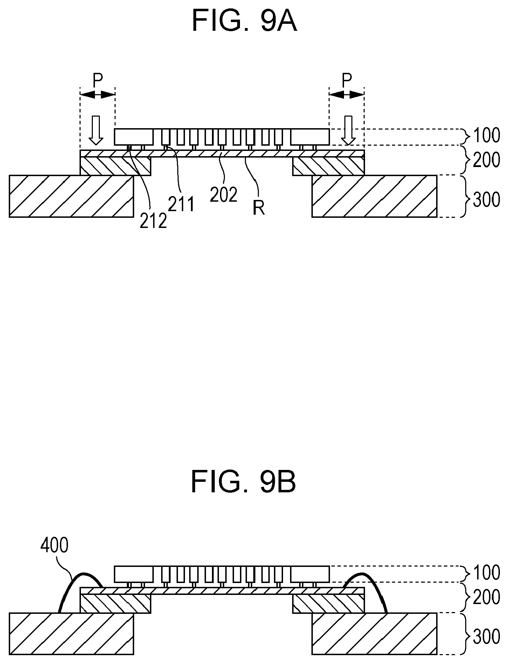

As illustrated in Fig. 8B, the mount substrate 300 is disposed so as to face the mirror substrate 200 that has a larger projection area than the actuator substrate 100. In addition, as illustrated in Fig. 9A, the mount substrate 300 is bonded to the mirror substrate 200 in a region P in which the mirror substrate 200 and the actuator substrate 100 do not overlap each other in an in-plane direction parallel to the in-plane direction of the reflective member 202. Accordingly, similar effects to those of the first exemplary embodiment can be obtained.

The present exemplary embodiment differs from the first exemplary embodiment in the electrical connection configuration of the drive circuit and the actuators 101. Hereinafter, the difference from the configuration of the first exemplary embodiment will be described.

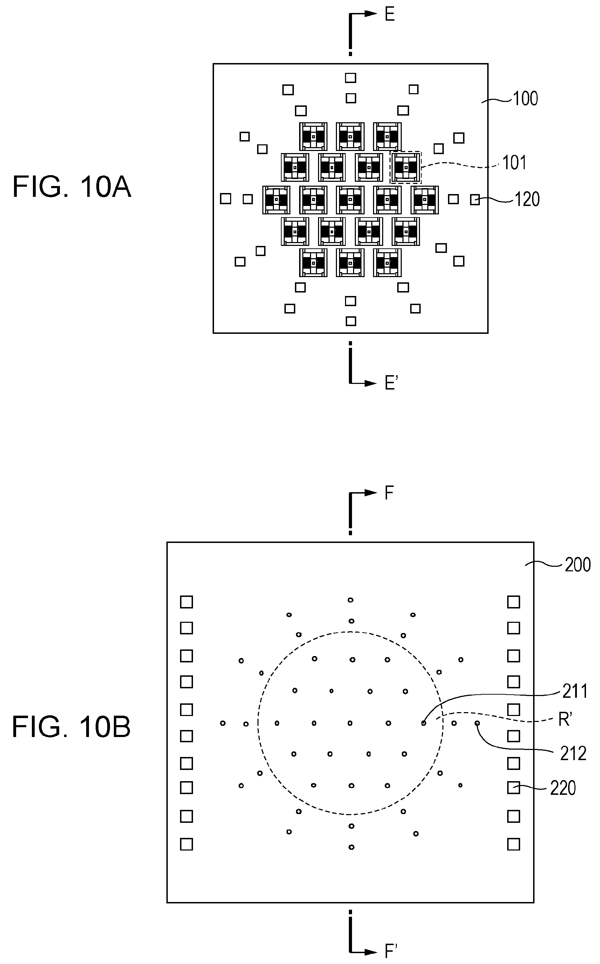

Fig. 10A schematically illustrates an example of the actuator substrate 100 according to the present exemplary embodiment, as viewed in the +Z direction, with the mirror substrate 200 and the mount substrate 300 omitted. A sectional view taken along the line E-E' indicated in Fig. 10A corresponds to the sectional view illustrated in Fig. 8B. Fig. 10B schematically illustrates an example of the mirror substrate 200 according to the present exemplary embodiment, as viewed in the -Z direction, with the actuator substrate 100 and the mount substrate 300 omitted. A sectional view taken along the line F-F' indicated in Fig. 10B corresponds to the sectional view illustrated in Fig. 8B.

As illustrated in Fig. 8B, the reflective member 202 of the mirror substrate 200 is bonded to the actuators 101 with bonding portions 211 interposed therebetween. In addition, the actuator substrate 100 is bonded to the reflective member 202 with bonding portions 212 (see Fig. 10B) interposed therebetween in bonding regions 120 (see Fig. 10A) in which the actuator substrate 100 is bonded to the reflective member 202 in regions other than where the actuators 101 are located. The bonding regions 120 may be located so as to correspond to regions other than the region where the reflective member 202 of the mirror substrate 200 is exposed.

The mirror substrate 200 includes the bonding portions 211 that are to be bonded to the actuators 101, the bonding portions 212 that are to be bonded to the bonding regions 120 of the actuator substrate 100, and conductive members 220. The bonding portions 211 have conductive properties, and the conductive members 220 are electrically connected to the bonding portions 211 by electric wires (not illustrated) formed in the mirror substrate 200. In addition, the bonding wires 400 illustrated in Fig. 8B electrically connect the conductive members 220 to the drive circuit of the mount substrate 300.

Thus, the drive circuit of the mount substrate 300 is electrically connected to the actuators 101 with the bonding wires 400, the conductive members 220, the wires (not illustrated) in the mirror substrate 200, and the bonding portions 211 interposed therebetween. Each of the actuators 101 is electrically connected to a corresponding one of the conductive members 220. Specifically, as illustrated in Fig. 10A, the actuator substrate 100 includes nineteen actuators 101. Meanwhile, as illustrated in Fig. 10B, the mirror substrate 200 has twenty conductive members 220 formed thereon. Nineteen of the twenty conductive members 220 drive the movable comb electrodes 104 in the actuators 101 (see Fig. 4B) for the respective actuators 101. The remaining one of the conductive members 220 provides a common reference potential to the fixed comb electrodes 105 of the respective nineteen actuators 101 (see Fig. 4B).

The conductive bonding portions 211 on the mirror substrate 200 are, for example, Au stud bumps each having a bump diameter of 35 $\mu$m and a height of 40 $\mu$m, for example. The bonding portions 211 may be Au bumps formed through an Au electrolytic plating technique, or any other material that has conductive properties can be used. The dimensions of each bonding portion 211 are not limited to the aforementioned values. A diameter that is too small may lead to a decrease in the bonding strength, whereas a diameter that is too large may affect the shape of the mirror. Accordingly, the diameter of each bonding portion 211 is preferably no less than 20 $\mu$m and no greater than 50 $\mu$m, and the height of each bonding portion 211 is preferably no less than 20 $\mu$m and no greater than 50 $\mu$m.

In addition, since the electrical connection configuration described above is used in the present exemplary embodiment, unlike the first exemplary embodiment, bonding wires are not provided between the mirror substrate 200 and the actuator substrate 100, as illustrated in Fig. 8B. Thus, in the mounting process illustrated in Fig. 9B, a process of attaching bonding wires to the actuator substrate 100 is rendered unnecessary. Accordingly, in the mounting process as well, an external force is not exerted on the bonding portions 211 and 212, and deformation of the bonding portions 211 and 212 can thus be suppressed.

Although the actuator substrate 100 and the mirror substrate 200 are rectangular in shape along the XY plane in the present exemplary embodiment as illustrated in Fig. 8C, the actuator substrate 100 and the mirror substrate 200 may be circular in shape as in the first exemplary embodiment.

Third Exemplary Embodiment

In the present exemplary embodiment, unlike the second exemplary embodiment, electrical connection to the actuators 101 is achieved not through the bonding portions 211 that are to be bonded to the actuators 101 but through the bonding portions 212 that are bonded to the actuator substrate 100 in regions other than where the actuators 101 are provided. Other configurations are similar to those of the second exemplary embodiment.

The bonding regions 120 can be constituted by metal films, such as Au thin films. The bonding regions 120 each have dimensions of 40 $\mu$m on each side and a thickness of 300 nm, for example. The dimensions are not limited to such values.

In addition, the bonding portions 212 have conductive properties and are, for example, Au stud bumps each having a bump diameter of 35 $\mu$m and a height of 40 $\mu$m, for example. The bonding portions 212 may be Au bumps formed through an Au electrolytic plating technique, or any other material that has conductive properties can be used.

The dimensions of each bonding portion 212 are not limited to the aforementioned values. A diameter that is too small may lead to a decrease in the bonding strength, whereas a diameter that is too large may affect the shape of the mirror. Accordingly, the diameter of each bonding portion 212 is preferably no less than 20 $\mu$m and no greater than 50 $\mu$m, and the height of each bonding portion 212 is preferably no less than 20 $\mu$m and no greater than 50 $\mu$m.

The bonding regions 120 on the actuator substrate 100 and the bonding portions 212 on the mirror substrate 200 are positioned relative to each other and are then bonded, for example, through an Au-Au surface-activated bonding technique.

In the present exemplary embodiment, each actuator 101 is electrically connected to a corresponding one of the bonding regions 120 on the actuator substrate 100 by wires (not illustrated) formed in the actuator substrate 100. Then, the bonding regions 120 are electrically connected to the bonding portions 212 on the mirror substrate 200, and the bonding portions 212 are electrically connected to the conductive members 220 by electric wires (not illustrated) formed in the mirror substrate 200. In addition, the bonding wires 400 illustrated in Fig. 8B electrically connect the conductive members 220 to the drive circuit of the mount substrate 300. Thus, the drive circuit is electrically connected to the actuators 101 with the bonding wires 400, the conductive members 220, the wires (not illustrated) in the mirror substrate 200, the bonding portions 212, the bonding regions 120, and the wires (not illustrated) in the actuator substrate 100 interposed therebetween.

As illustrated in Fig. 10A, the number of the actuators 101 is 19, whereas the number of the bonding regions 120 and the number of the bonding portions 212 are both 24. Each of the nineteen actuators 101 is connected to a corresponding one of the nineteen bonding regions 120 (and the bonding portions 212) out of the twenty-four bonding regions 120 (and the bonding portions 212). This is for driving the movable comb electrodes 104 in the actuators 101 (see Fig. 4B) for the respective actuators 101. Meanwhile, one bonding region 120 out of the remaining five bonding regions 120 and one bonding portion 212 out of the remaining five bonding portions 212 provide a common reference potential to the fixed comb electrodes 105 in the nineteen actuators 101 (see Fig. 4B). The remaining four bonding regions 120 and the remaining four bonding portions 212 are formed with the rotational symmetry of the bonding regions 120 and the bonding portions 212 along the plane in which they are bonded taken into consideration. These remaining four bonding regions 120 and these remaining four bonding portions 212 are not electrically connected to the actuators 101.

The above-described configuration makes it unnecessary to provide wiring in a region corresponding to the face at which the reflective surface R of the reflective member 202 of the mirror substrate 200 is exposed, and an influence of such wiring on the deformation of the reflective surface R can be suppressed.

Fourth Exemplary Embodiment

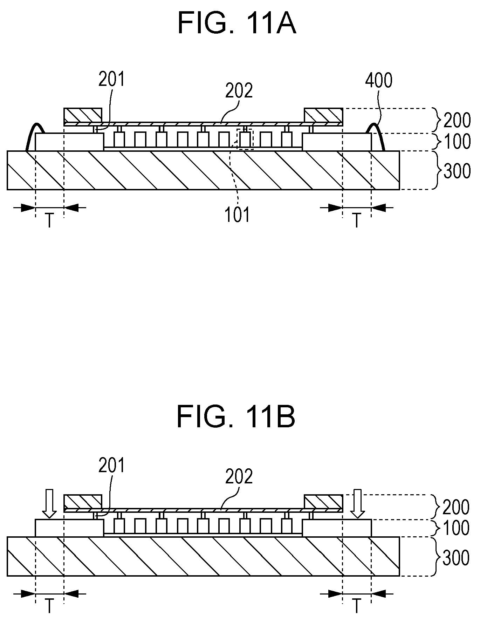

Figs. 11A schematically illustrates a deformable mirror according to the present exemplary embodiment. In the present exemplary embodiment, unlike the first exemplary embodiment, the projection area of the actuator substrate 100 is larger than the projection area of the mirror substrate 200. Along with this configuration, the mount substrate 300 is disposed so as to face the actuator substrate 100 that has a larger projection area than the mirror substrate 200. The mount substrate 300 is bonded to the actuator substrate 100 in a region T in which the mirror substrate 200 and the actuator substrate 100 do not overlap each other in an in-plane direction parallel to the in-plane direction of the reflective member 202.

In this configuration as well, as illustrated in Fig. 11B, an external force can be exerted on the actuator substrate 100 (as indicated by the arrows) in the region T in which the mirror substrate 200 and the actuator substrate 100 do not overlap each other in an in-plane direction parallel to the in-plane direction of the reflective member 202. Thus, the external force can be exerted directly on the actuator substrate 100 and the mount substrate 300 without involving the bonding portions 201. Accordingly, deformation of the bonding portions 201 can be suppressed.

The mount substrate 300 is bonded to the actuator substrate 100 so as not to make contact with the plurality of actuators 101. This is for securing a wider movable range of the actuators 101. Specifically, as illustrated in Fig. 11A, the actuator substrate 100 is spaced apart from the mount substrate 300 in a region in which the plurality of actuators 101 are disposed.

In addition, the drive circuit (not illustrated) of the mount substrate 300 is electrically connected to the actuators 101 on the actuator substrate 100 by the bonding wires 400. The configuration of the actuators 101 is the same as that of the first exemplary embodiment.

Fifth Exemplary Embodiment

An adaptive optical system in which a deformable mirror according to any one of the first to fourth exemplary embodiments is used as a wavefront correction device for compensating for the optical aberration (wavefront aberration) generated in an optical path will be described hereinafter. Specifically, the description will be given through an example in which the adaptive optical system is applied in a scanning laser ophthalmoscope (hereinafter, referred to as an SLO apparatus), which is a type of an ophthalmologic apparatus. An SLO apparatus irradiates a fundus with light so as to enable the observation of visual cells, retina nerve fiber bundles, blood cell kinetics, and so on.

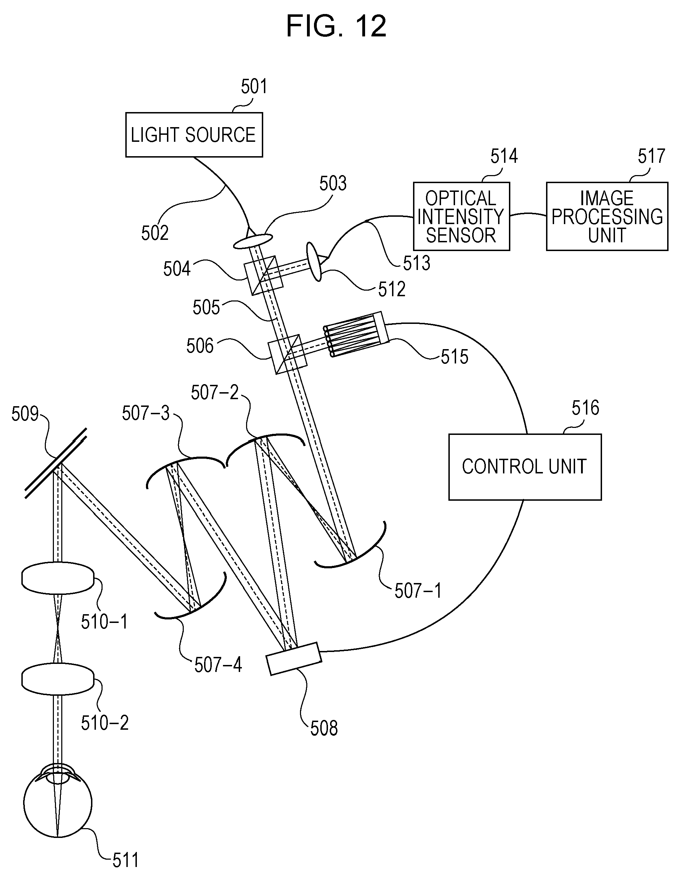

Fig. 12 schematically illustrates the configuration of an SLO apparatus according to the present exemplary embodiment. Light emitted by a light source 501 propagates through a single-mode optical fiber 502 and is collimated upon passing through a collimator 503. The collimated light rays are transmitted through a beam splitter 504 serving as an optical splitting unit and are then guided, as measurement light 505, to an adaptive optical system. The wavelength of the light source 501 is not particularly limited, and light at a wavelength in a range from approximately 800 nm to 1500 nm is suitably used for capturing a fundus image, while suppressing a dazzling effect on the subject and maintaining the resolution.

The adaptive optical system includes a beam splitter 506 serving as an optical splitting unit, a wavefront sensor (acquisition unit) 515, a deformable mirror (reflective optical modulation unit) 508 provided with a mirror unit having a reflective surface, and reflective mirrors 507-1 to 507-4 for guiding the light to the beam splitter 506, the wavefront sensor 515, and the deformable mirror 508. The reflective mirrors 507-1 to 507-4 are disposed such that at least the pupil of an eye 511 to be examined, the wavefront sensor 515, and the deformable mirror 508 have an optically conjugate relationship.

The light that has passed through the adaptive optical system is scanned one-dimensionally or two-dimensionally by an optical scanning unit 509. The measurement light scanned by the optical scanning unit 509 passes through eyepiece lenses 510-1 and 510-2 and enters the eye 511. Optimal irradiation of the eye 511 can be achieved in accordance with the visibility of the eye 511 by adjusting the positions of the eyepiece lenses 510-1 and 510-2. Although the lenses are used in an eyepiece portion, a spherical mirror or the like may instead be used.

The measurement light that has entered the eye 511 is either reflected or scattered by the fundus (retina). The reflection light that has been reflected or scattered by the fundus of the eye 511 travels backward through the path that the light has traveled to enter the eye 511, and some of the light is reflected by the beam splitter 506 and enters the wavefront sensor 515, in which the light is used to measure the wavefront. The wavefront sensor 515 can be constituted by a known Shack-Hartmann wavefront sensor.

Part of the reflected or scattered light that has passed through the beam splitter 506 is partially reflected by the beam splitter 504, and the reflected part of the light is guided to and received by an optical intensity sensor 514 through a collimator 512 and an optical fiber 513. The light that has entered the optical intensity sensor 514 is converted to an electric signal, and the electric signal is processed to form a fundus image by an image processing unit 517.

The wavefront sensor 515 is connected to a control unit 516. The wavefront sensor 515 acquires information on the wavefront of the received light rays and transmits the information to the control unit 516. The control unit 516 is connected to the deformable mirror 508 and causes the deformable mirror 508 to deform into a shape as instructed by the control unit 516.

The control unit 516 computes a mirror shape that corrects the wavefront to a wavefront without aberration on the basis of the wavefront information acquired from the wavefront sensor 515. The control unit 516 then calculates differences among the voltages to be applied to the respective comb electrodes that are necessary to reproduce the computed shape in the deformable mirror 508, and transmits the calculated voltage differences to the deformable mirror 508. The deformable mirror 508 applies the voltages across the movable comb electrodes and the fixed comb electrodes through the drive circuit of the mount substrate in accordance with the voltage differences transmitted from the control unit 516 so as to cause the mirror surface to deform into a predetermined shape. The measurement of the wavefront by the wavefront sensor 515, the transmission of the wavefront information to the control unit 516, and the instruction on the correction of the aberration from the control unit 516 to the deformable mirror 508 described above are iteratively processed, and thus feedback control for retaining an optimal wavefront is carried out.

With the use of the adaptive optical system according to the present exemplary embodiment, the deformable mirror 508 can be deformed in a broad range, and the aberration can be compensated for in a broad range. In addition, the adaptive optical system makes it possible to quickly respond to an instruction from the control unit 516 so as to compensate for the aberration. Accordingly, the SLO apparatus that includes the adaptive optical system according to the present exemplary embodiment makes it possible to appropriately compensate for the aberration generated in an eye to be examined, and a high-resolution image can thus be obtained.

According to some of the exemplary embodiments of the present invention, deformation of bonding portions in a deformable mirror provided with a mount substrate (third substrate) can be suppressed.

While the present invention has been described with reference to exemplary embodiments, it is to be understood that the invention is not limited to the disclosed exemplary embodiments. The scope of the following claims is to be accorded the broadest interpretation so as to encompass all such modifications and equivalent structures and functions.

**Claims**

1. A method for fabricating a deformable mirror that includes a first substrate (100) provided with a plurality of actuators (101), a second substrate (200) provided with a reflective member (202) that is bonded to the plurality of actuators with bonding portions (201) interposed therebetween, and a third substrate (300) provided with a drive circuit for driving the plurality of actuators, the first and second substrates having respective projection areas defined by outer peripheries of shadows of the first and second substrates obtained when the shadows are projected on a plane parallel to an in-plane direction of the reflective member, the method comprising:

   a step of preparing the first substrate provided with the plurality of actuators and the second substrate provided with the reflective member and bonded to the first substrate with the bonding portions interposed therebetween;
   a step of disposing the third substrate provided with the drive circuit for driving the plurality of actuators so as to face one of the first substrate and the second substrate that has a larger projection area; and
   a step of bonding the one of the first substrate and the second substrate that has a larger projection area to the third substrate in a region in which the first substrate and the second substrate do not overlap each other in an in-plane direction that is parallel to the in-plane direction of the reflective member.

2. The method for fabricating the deformable mirror according to claim 1, wherein the one of the first substrate and the second substrate that has a larger projection area is the second substrate.

3. The method for fabricating the deformable mirror according to claim 2, wherein the third substrate includes an opening through which a reflective surface of the reflective member is exposed.

4. The method for fabricating the deformable mirror according to claim 1, wherein the one of the first substrate and the second substrate that has a larger projection area is the first substrate.

5. A deformable mirror, comprising:

   a first substrate (100) provided with a plurality of actuators (101);
   a second substrate (200) provided with a reflective member (202) that is bonded to the plurality of actuators with a bonding portion (201) interposed therebetween; and
   a third substrate (300) provided with a drive circuit for driving the plurality of actuators,
   wherein, provided that an area defined by an outer periphery of a shadow of the first or second substrate obtained when the shadow is projected on a plane parallel to an in-plane direction of the reflective member is termed a projection area,
   the third substrate faces one of the first substrate and the second substrate that has a larger projection area, and is bonded to the one of the first substrate and the second substrate that has a larger projection area.

6. The deformable mirror according to claim 5, wherein the third substrate is bonded to the one of the first substrate and the second substrate that has a larger projection area in a region in which the first substrate and the second substrate do not overlap each other in an in-plane direction parallel to the in-plane direction of the reflective member.

7. The deformable mirror according to claim 5 or 6, wherein the one of the first substrate and the second substrate that has a larger projection area is the second substrate.

8. The deformable mirror according to claim 7, wherein the third substrate includes an opening through which a reflective surface of the reflective member is exposed.

9. The deformable mirror according to claim 7 or 8, wherein the drive circuit is electrically connected to the actuators with a conductive member formed in the second substrate interposed therebetween.

10. The deformable mirror according to claim 9, wherein the conductive member is electrically connected to the actuators by a first bonding wire, and wherein the conductive member is electrically connected to the drive circuit by a second bonding wire.

11. The deformable mirror according to claim 9, wherein the bonding portion has conductive properties, and wherein the drive circuit is electrically connected to the actuators with the conductive member formed in the second substrate, the bonding portion, and a bonding wire interposed therebetween.

**12.** The deformable mirror according to claim 9, wherein the second substrate includes a first bonding portion that has conductive properties and that is to be bonded to the first substrate in a region other than where the plurality of actuators are provided, and
wherein the drive circuit is electrically connected to the actuators with the conductive member formed in the second substrate, the first bonding portion, and a bonding wire interposed therebetween.

**13.** The deformable mirror according to claim 5 or 6, wherein the one of the first substrate and the second substrate that has a larger projection area is the first substrate.

**14.** The deformable mirror according to claim 13, wherein the third substrate is bonded to the first substrate such that the third substrate does not make contact with the plurality of actuators.

**15.** An optical system, comprising:

a reflective optical modulation unit configured to correct a wavefront aberration of light incident thereon;
an acquisition unit configured to acquire information on a wavefront of light incident thereon; and
a control unit configured to control the reflective optical modulation unit on the basis of the information on the wavefront acquired by the acquisition unit,
wherein the reflective optical modulation unit includes the deformable mirror according to any one of claims 5 to 14.

FIG. 1A

FIG. 1B

FIG. 1C

# FIG. 2A

# FIG. 2B

# FIG. 3A

# FIG. 3B

FIG. 4A

101

102

100

Z → Y

X

FIG. 4B

108b

104

105

IVC

IVC

106

108a

107

Z → Y

X

FIG. 4C

108a  105  104  106  107  108b

100

FIG. 5A

FIG. 5B

FIG. 5C

FIG. 5D

EP 3 028 626 A1

FIG. 6A

FIG. 6B

FIG. 6C

FIG. 6D

FIG. 6E

FIG. 6F

FIG. 6G

FIG. 6H

# FIG. 7A

# FIG. 7B

FIG. 8A

300
200
R

VIIIB                                    VIIIB

Y
Z⊙——X

FIG. 8B

211    212

R  202

400                    E          E'       300
F          P              P   F'              200
                                             100

Z
Y⊗——X

FIG. 8C

Z⊗——X
Y

L1
L2
S2
S1

# FIG. 9A

# FIG. 9B

FIG. 10A

FIG. 10B

# FIG. 11A

# FIG. 11B

# FIG. 12

501 LIGHT SOURCE

502

503

504

505

506

507-1

507-2

507-3

507-4

508

509

510-1

510-2

511

512

513

514 OPTICAL INTENSITY SENSOR

515

516 CONTROL UNIT

517 IMAGE PROCESSING UNIT

Europäisches
Patentamt

European
Patent Office

Office européen
des brevets

# EUROPEAN SEARCH REPORT

Application Number

EP 15 19 8061

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2008/225370 A1 (MANSELL JUSTIN DENNIS [US]) 18 September 2008 (2008-09-18) <br><br> * paragraphs [0045] - [0054]; figures 4-6 * <br> ----- | 1,2,5-7, 9-12,14, 15 | INV. A61B3/10 G02B7/182 G02B26/08 |
| X | WO 93/25929 A1 (UNITED TECHNOLOGIES CORP [US]) 23 December 1993 (1993-12-23) <br> * pages 1-13; figures 1,2 * <br> ----- | 1,5 | |
| X | EP 2 767 858 A1 (CANON KK [JP]) 20 August 2014 (2014-08-20) <br> * figures 1,3,5,12 * <br> ----- | 1,4-6, 9-15 | |
| X | US 2011/211190 A1 (BAE SYSTEMS PLC [GB]; GRIFFITH MICHAEL STEWART [GB]; LAYCOCK LESLIE CH) 1 September 2011 (2011-09-01) <br> * paragraphs [0076] - [0088]; figure 8 * <br> ----- | 1,4-6, 9-15 | |
| X | US 2011/211268 A1 (CAMET SEBASTIEN [FR] ET AL) 1 September 2011 (2011-09-01) <br> * figure 11 * <br> ----- | 1,5 | |
| X | US 2006/262434 A1 (EALEY MARK A [US] ET AL) 23 November 2006 (2006-11-23) <br> * figures 8,9 * <br> ----- | 1,2,5-7 | TECHNICAL FIELDS SEARCHED (IPC) <br><br> A61B <br> G02B |
| X | JP 2008 241738 A (TOPCON CORP) 9 October 2008 (2008-10-09) <br><br> * paragraphs [0005] - [0026]; figure 1 * <br> ----- | 1,3,5, 8-12,14, 15 | |
| A | US 2007/137989 A1 (DIVOUX CLAIRE [FR] ET AL) 21 June 2007 (2007-06-21) <br> * figure 1 * <br> ----- | 1,5 | |

The present search report has been drawn up for all claims

| Place of search | Date of completion of the search | Examiner |
|---|---|---|
| Munich | 5 April 2016 | Baur, Christoph |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

                              

& : member of the same patent family, corresponding
document

EPO FORM 1503 03.82 (P04C01)

## EP 3 028 626 A1

### ANNEX TO THE EUROPEAN SEARCH REPORT
### ON EUROPEAN PATENT APPLICATION NO.

EP 15 19 8061

This annex lists the patent family members relating to the patent documents cited in the above-mentioned European search report.
The members are as contained in the European Patent Office EDP file on
The European Patent Office is in no way liable for these particulars which are merely given for the purpose of information.

05-04-2016

| Patent document cited in search report | | Publication date | Patent family member(s) | | Publication date |
|---|---|---|---|---|---|
| US 2008225370 | A1 | 18-09-2008 | NONE | | |
| WO 9325929 | A1 | 23-12-1993 | US | 5357825 A | 25-10-1994 |
| | | | WO | 9325929 A1 | 23-12-1993 |
| EP 2767858 | A1 | 20-08-2014 | CN | 103995350 A | 20-08-2014 |
| | | | EP | 2767858 A1 | 20-08-2014 |
| | | | JP | 2014178675 A | 25-09-2014 |
| | | | US | 2014232984 A1 | 21-08-2014 |
| US 2011211190 | A1 | 01-09-2011 | AU | 2009316985 A1 | 27-05-2010 |
| | | | EP | 2359108 A2 | 24-08-2011 |
| | | | US | 2011211190 A1 | 01-09-2011 |
| | | | WO | 2010058193 A2 | 27-05-2010 |
| US 2011211268 | A1 | 01-09-2011 | CA | 2734107 A1 | 18-02-2010 |
| | | | DK | 2324387 T3 | 24-02-2014 |
| | | | EP | 2324387 A1 | 25-05-2011 |
| | | | FR | 2935054 A1 | 19-02-2010 |
| | | | JP | 5695565 B2 | 08-04-2015 |
| | | | JP | 2011530721 A | 22-12-2011 |
| | | | US | 2011211268 A1 | 01-09-2011 |
| | | | WO | 2010018326 A1 | 18-02-2010 |
| US 2006262434 | A1 | 23-11-2006 | US | 2006262434 A1 | 23-11-2006 |
| | | | WO | 2006121676 A1 | 16-11-2006 |
| JP 2008241738 | A | 09-10-2008 | NONE | | |
| US 2007137989 | A1 | 21-06-2007 | EP | 1697783 A1 | 06-09-2006 |
| | | | FR | 2864634 A1 | 01-07-2005 |
| | | | JP | 4558745 B2 | 06-10-2010 |
| | | | JP | 2007517252 A | 28-06-2007 |
| | | | US | 2007137989 A1 | 21-06-2007 |
| | | | WO | 2005069057 A1 | 28-07-2005 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6384952 B